# EUROPÄISCHE PATENTSCHRIFT

(11) **EP 1 418 170 B1**
(45) Veröffentlichungstag und Bekanntmachung des Hinweises auf die Patenterteilung: **07.06.2006**
(21) Anmeldenummer: 04002913.4
(22) Anmeldetag: 24.11.1998
(51) Int. Cl.: C07C 213/00, C07C 215/42, C07C 213/02, C07C 213/10, C07D 473/32, C12P 41/00, C12P 13/00, C07D 473/00, C07C 275/64

(54) **Verfahren zur Herstellung von optisch aktiven Aminoalkoholen durch Racematspaltung**
Process for the preparation of optically active amino alcohols by optical resolution
Procédé de préparation d' aminoalcools optiquement actifs par résolution optique

(30) Priorität: 27.11.1997 CH 273997; 03.12.1997 CH 278197; 21.01.1998 CH 13398; 27.03.1998 CH 72398; 07.10.1998 EP 98118895
(43) Veröffentlichungstag der Anmeldung: 12.05.2004
(62) Teilanmeldung aus: 98122293.8
(73) Patentinhaber: Lonza AG, 3930 Visp (CH)
(72) Erfinder: Berchtold Katja, 3937 Baltschieder (CH); Roduit, Jean-Paul, Dr., 1950 Sion (CH); Bernegger-Egli, Christine, Dr., 3985 Münster (CH); Urban, Eva Maria, 3912 Termen (CH); Petersen, Michael, Dr., 79540 Lörrach (DE)
(74) Vertreter: Winter, Brandl, Fürniss, Hübner Röss, Kaiser, Polte Partnerschaft Patent- und Rechtsanwaltskanzlei

(56) Entgegenhaltungen:
- EP-A- 0 434 450
- EP-A- 0 878 548
- WO-A-93/17020
- WO-A-95/21161
- WO-A-97/45529
- WO-A-99/19327
- EVANS C T ET AL: "POTENTIAL USE OF CARBOCYCLIC NUCLEOSIDES FOR THE TREATMENT OF AIDS CHEMO-ENZYMATIC SYNTHESES OF THE ENANTIOMERS OF CARBOVIR" JOURNAL OF THE CHEMICAL SOCIETY, PERKIN TRANSACTIONS 1, CHEMICAL SOCIETY. LETCHWORTH, GB, Nr. 5, 1992, Seiten 589-592, XP002089871 ISSN: 0300-922X
- CAMPBELL, JEFFREY A. ET AL: "Chirospecific Syntheses of Precursors of Cyclopentane and Cyclopentene Carbocyclic Nucleosides by [3 + 3]-Coupling and Transannular Alkylation" J. ORG. CHEM. (1995), 60(14), 4602-16 CODEN: JOCEAH;ISSN: 0022-3263, XP002041314
- N. KATAGIRI ET AL.: "Deamination of 9-(hydroxymethylated cycloalkyl)-9H-adenines (carbocyclic adenine nucleosides) by adenosine deaminase: Effect of the high-pressure upon deamination rate and enantioselectivity" NUCLEOSIDES & NUCLEOTIDES., Bd. 15, Nr. 1-3, 1996, Seiten 631-647, XP002124310 MARCEL DEKKER, INC., US ISSN: 0732-8311
- J.R. MALPASS ET AL.: "Reaction of Chlorosuphonyl Isocyanate with 1,3-Dienes. Control of 1,2- and 1,4-Addition Pathways and the Synthesis of Aza- and Oxa-bicyclic Systems" JOURNAL OF THE CHEMICAL SOCIETY, PERKIN TRANSACTIONS 1, 1977, Seiten 874-884, XP002104986 LETCHWORTH GB

## Beschreibung

Die vorliegende Erfindung betrifft ein neues Verfahren zur Herstellung von (1R,4S)- oder (1S,4R)-1-Amino-4-(hydroxymethyl)-2-cyclopenten der Formeln 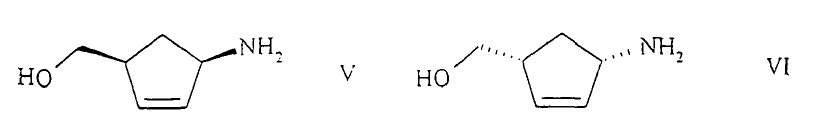 deren Salzen, oder deren D- oder L-Hydrogentartraten sowie deren Weiterumsetzung zu (1S,4R)- oder (1R,4S)-4-(2-Amino-6-chlor-9-H-purin-9-yl)-2-cyclopenten. (1R,4S)-1-Amino-4-(hydroaymethyl)-2-cyclopenten der Formel IV ist ein wichtiges Zwischenprodukt zur Herstellung von carbocyclischen Nukleosiden wie z. B. Carbovir® (Campbell et al., J. Org. Chem 1995, 60, 4602 - 4616)

Ein Verfahren zur Herstellung von (1R,4S)-1-Amino-4-(hydroxymethyl)-2-cyclopenten ist beispielsweise von Campbell et al (ibid) und von Park K H. & Rapoport H. (J. Org. Chem. 1994, 59, 394 - 399) beschrieben
Bei diesem Verfahren dient als Edukt entweder D-glucon-δ-lacton oder D-Serin, wobei ca. 15 Synthesestufen bis zur Bildung von (1R,4S)-N-tert-Butoxycarbonyl-4-hydroxymethyl-2-cyclopenten, welches dann zum (1R,4S)-1-Amino-4-(hydroxymethyl)-2-cyclopenten deprotelttioniert wird, notwendig sind
Diese beiden Verfahren sind kostspielig, umständlich und grosstechnisch nicht gangbar.
Die WO 93/17020 beschreibt ein Verfahren zur Herstellung von (1R,4S)-1-Amino-4-(hydroxymethyl)-2-cyclopenten, wobei (1R,4S)-4-Amino-2-cyclopenten-1-carbonsäure mit Lithiumaluminiumhydrid zum gewünschten Produkt reduziert wird.
Nachteilig bei diesem Verfahren ist zum einen, dass auch die Doppelbindung des Cyclopentenringes reduziert wird, die schlechte Handhabbarkeit von Lithiumaluminiumhydrid und zum anderen, dass es zu kostspielig ist.
Taylor S. J. et al. (Tetrahetron: Asymmetry Vol. 4, No. 6, 1993, 1117 - 1128) beschreiben ein Verfahren zur Herstellung von (1R,4S)-1-Amino-4-(hydroxymethyl)-2-cyclopenten ausgehend von (±)-2-Azabicyclo[2.2.1]hept-5-en-3-on als Edukt Dabei wird das Edukt mittels Mikroorganismen der Spezies Pseudomonas solanacearum oder Pseudomonas fluorescens in (1R,4S)-2-Azabicyclo[2.2.1]hept-5-en-3-on überführt, welches dann mit Di-tert-butyldicarbonat in das (1R,4S)-N-tert-butoxycarbonyl-2-azabicyclo[2.2.1]hept-5-en-3-on umgesetzt wird. Letzteres wird mit Natriumborhydrid und Trifluoressigsäure zum gewünschten Produkt reduziert. Auch ist dieses Verfahren viel zu kostspielig.

Desweiteren beschreiben Martinez et al. (J. Org. Chem. 1996, 61, 7963 - 7966) eine 10stufige Synthese von (1R,4S)-1-Amino-4-(hydroxymethyl)-2-cyclopenten ausgehend von Dialkylmalonsäurediethylester. Auch dieses Verfahren hat den Nachteil, dass es umständlich und grosstechnisch nicht gangbar ist.
Bekannt ist auch, dass man N-substituierte-(±)-2-Azabicyclo[2.2.1]hept-5-en-3-one, welche einen elektronenziehenden Substituenten tragen, mit einem Metallhydrid zu den entsprechenden N-substituierten Aminoalkoholen reduzieren kann (Katagiri et al., Tetrahedron Letters, 1989, 30, 1645 - 1648; Taylor et al., ibid).
Im Gegensatz dazu ist bekannt, dass unsubstituiertes (±)-2-Azabicyclo[2.2.1]hept-5-en-3-on der Formel 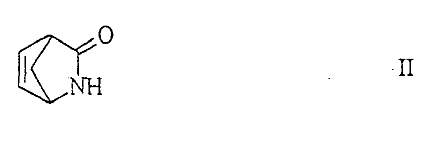 mit Lithiumaluminiumhydrid zu-(±)-2-Azabicyclo[2.2.1]octen reduziert wird (Malpass & Tweedle, J. Chem. Soc., Perkin Trans 1, 1977, 874 - 884) und dass die direkte Reduktion von (±)-2-Azabicyclo[2.2.2]hept-5-en-3-on zum entsprechenden Aminoalkohol bislang als nicht möglich galt (Katagiri et al., ibid; Taylor et al., ibid).

Bekannt ist auch, racemisches 1-Amino-4-(hydroxymethyl)-2-cyclopenten mittels (-)- Dibenzoylweinsäure zu spalten (US-A 034 394). Diese Umsetzung hat einerseits den Nachteil, dass die (-)-Dibenzoylweinsäure teuer ist, anderseits, dass die Trennung in Gegenwart eines genau definierten Gemisches von Acetonitril und Ethanol erfolgen muss. Dieses Lösungsmittetgemisch kann nicht getrennt werden und muss der Verbrennung zugeführt werden.

Aufgabe der vorliegenden Erfindung war es, ein einfaches, ökonomisches und kostengünstiges Verfahren zur Herstellung von (1R,4S)-1-Amino-4-(hydroxymethyl)-2-cyclopenten zur Verfügung zu stellen, welche hrerseits in mehrstufigen Synthesen verwendet werden können.

Diese Aufgabe wurde entsprechend Anspruch 1 gelöst, indem der racemische, bevorzugt der cis-racemische, Aminoalkohol der Formel I mit einer optisch aktiven Weinsäure in einer chemischen Racematspaltung in das (1R,4S)- oder (1S,4R)-1-Amino-4-(hydroxymethyl)-2-cyclopenten der Formel 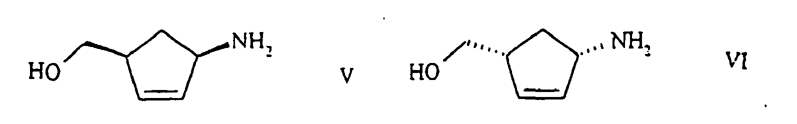 bzw. deren Salze umgesetzt wird.

Die chemische Racematspaltung wird mit einer optisch aktiven Weinsäure wie mit D-(-)-Weinsäure oder L-(+)-Weinsäure durchgeführt.

Die Racematspaltung mit D-(-)-Weinsäure wird zweckmässig so durchgeführt, dass das racemische 1-Amino-4-(hydroxymethyl)-2-cyclopenten zunächst mit der D-(-)-Weinsäure in Gegenwart eines C₁₋₆-Alkohols zur Umsetzung gebracht wird.
Geeignete C₁₋₆-Alkohole sind die gleichen wie die zuvor beschriebenen. Bevorzugt wird Methanol eingesetzt.
Die Umsetzung, die zur Salzbildung führt, wird üblicherweise bei einer Temperatur zwischen 20°C und Rückflusstemperatur des Lösungsmittels, vorzugsweise bei Rückflusstemperatur durchgeführt.
Wenn gewünscht, kann das hierbei entstehende 1-Amino-4-(hydroxymethyl)-2-cyclopenten-D-tartrat durch Umkristallisation aus einem C₁₋₆-Alkohol wie Methanol weiter gereinigt werden.

Die Racematspaltung mit L-(+)-Weinsäure wird zweckmässig analog zu der mit D-(-)-Weinsäure durchgeführt. D.h. dass die Racematspaltung mit L-(+)-Weinsäure ebenfalls in Gegenwart eines C₁₋₆-Alkohols und bei einer Temperatur zwischen 20 °C und Rückflusstemperatur des Lösungsmittels, vorzugsweise bei Rückflusstemperatur durchgerührt werden kann. Nach Abkühlen kristallisiert das (1S,4R)-1-Amino-4-(hydroxymethyl)-2-cyclopenten-hydrogen-L-tartrat aus.
Das (1R,4S)-1-Amino-4-(hydroxymethyl)-2-cyclopenten hydrogen-L-tartrat liegt insbesondere gelöst in der Mutterlauge vor.
Die lsolatiorr, die weitere Reinigung (Freisetzung) sowie die Umsetzung zum, entsprechenden Salz des (1R,4S)- oder (1S,4R)-1-Amino-4-(hydroxymethyl)-2-cyclopentens erfolgt mit einer Base und anschliessender Säurebehandlung. Geeignete Basen sind Alkalimetallalkoholate, Alkalimetall- oder Erdalkalimetallcarbonate und Alkallmetall- oder Erdalkalimetallhydroxide. Als Alkalimetallalkoholate können Natrium- oder Kaliumalkoholate eingesetzt werden. Als Alkalimetallcarbonat kann Kalium- oder Natriumcarbonat, Kalium- oder Natriumhydrogen-carbonat und als Erdalkalimetallcarbonat kann Magnesium- oder Calciumcarbonat eingesetzt werden. Als Alkalimetallhydroxid kann Natrium- oder Kaliumhydroxid und als Erdalkalimetallhydroxid kann Calciumhydroxid verwendet werden. Die Umsetzung zum entsprechenden Salz erfolgt üblicherweise mit einer Mineralsäure wie mit Schwefelsäure, Salzsäure oder Phosphorsäure, vorzugsweise mit Salzsäure.

Das (1R,4S)- oder (1S,4R)-1-Amino-4-(hydroxymethyl)-2-cyclopenten hydrogen-D-tartrat und das (1R,4S)- oder (1S,4R)-1-Amino-4-(hydroxymethyl)-2-cyclopenten hydrogen-L-tartrat sind als nicht literaturbekannte Verbindungen ebenfalls Gegenstand der Erfindung.

Bevorzugt wird die chemische Racematspaltung mit D-(+)-Weinsäure wegen der höheren Leistung, technischen Leichtigkeit und effizienteren Racematspaltung durchgeführt.

Analog zum racemischen Aminoalkohol können selbstverständlich auch die optisch aktiven (1R,4S)- oder (1S,4R)-1-Amino-4-(hydroxymethyl)-2-cyclopentene mit D-(-)- oder L-(+)-Weinsäure zu den entsprechenden Tartraten umgesetzt werden.

Überraschenderweise wurde nun gefunden, dass wenn man (±)-2-Azabicyclo[2.2.1]hept-5-en-3-on der Formel 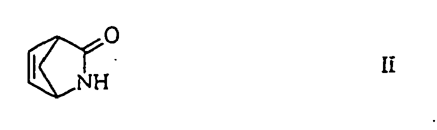 in Form des Racemats oder eines seiner optisch aktiven Isomere, mit einem Metallhydrid reduziert, der Aminoalkohol der Formel 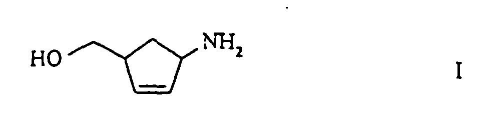 in Form des Racemats oder eines seiner optisch aktiven Isomere, auf einfache Weise erhalten wird.

Vorzugsweise wird der racemische cis-Aminoalkohol der Formel I erhalten.

Wie vorstehend beschrieben, kann der racemische Aminoalkohol der Formel I in einer chemischen Racematspaltung in das (1R,4S)- oder (1S,4R)-1-Amino-4-(hydroxymethyl)-2-cyclopenten der Formel V oder VI bzw. deren Salze umgesetzt werden.

Ein weiterer Bestandteil der vorliegenden Erfindung ist die Weiterumsetzung, die Acylierung, der (1R,4S)- oder (1S,4R)-1-Amino-4-(hydroxymethyl)-2-cyclopentene zu den (1R,4S)- oder (1S, 4R)-1-Amino-4-(hydroxymethyl)-2-cyclopentenderivaten der allgemeinen Formeln 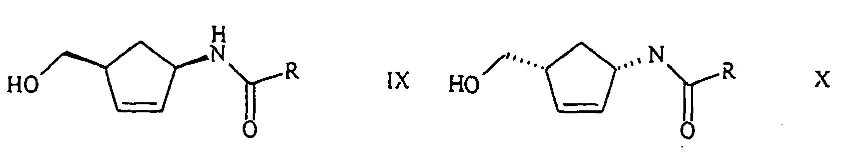 Worin R C₁₋₄-Alkyl, C₁₋₄-Alkoxy, Aryl Oder Aryloxy bedeutet.

C₁₋₄-Alkyl kann substituiert oder unsubstituiert sein. Unter substituiertem C₁₋₄-Alkyl wird im folgenden mit einem oder mehreren Halogenatomen substituiertes C₁-₄-Alkyl verstanden. Als Halogenatom kann F, Cl, Br oder J angewendet werden. Beispiele für C₁-₄-Alkyl sind Methyl, Ethyl, Propyl, Butyl, Isobutyl, tert. Butyl, Isopropyl, Chlormethyl, Brommethyl, Dichlormethyl, Dibrommethyl. Vorzugsweise wird als C₁-₄-Alkyl Methyl, Ethyl, Propyl, Butyl, Isobutyl oder Chlormethyl verwendet.

Als C₁-₄-Alkoxy kann beispielsweise Methoxy, Ethoxy, Propoxy oder Butoxy verwendet werden. Als Aryl kann beispielsweise Phenyl oder Benzyl, substituiert oder unsubstituiert, verwendet werden. Als substituiertes Phenyl oder Benzyl wird im folgenden mit einem oder mehreren Halogenatomen substituiertes Phenyl oder Benzyl verstanden, wie bspw.
Chlorbenzyl, Dichlorbenzyl, Bromphenyl oder Dibromphenyl.
Als Aryloxy kann beispielsweise Benzyloxy oder Phenoxy, substituiert mit den zuvor beschriebenen Substituenten oder unsubstituiert, verwendet werden.

Wie fachmännisch üblich kann der Aminoalkohol der Formel I mit einer Säure in die entsprechenden Salze überführt werden, wie beispielsweise in Hydrohalogenidsalze. Als Hydrohalogenidsalze sind Hydrobromide und Hydrochloride geeignet.

Das Edukt, das (±)-2-Azabicyclo[2.2.1]hept-5-en-3-on, kann gemäss der EP-A 0 508 352 hergestellt werden.

Als Metallhydride können Alkalimetall- oder Erdalkalimetallhydride sowie binäre oder komplexe Metallhydride der Bor- oder Aluminiumgruppe eingesetzt werden, beispielsweise Alkalimetall- und Erdalkalimetallborhydride und Alkalimetall- und Erdalkalimetallaluminiumhydride.

Als Alkalimetall- oder Erdalkalimetallhydride sind LiH, NaH, KH, BeH₂, MgH₂ oder CaH₂ geeignet. Als binäre Alkalimetall- oder Erdalkalimetallborhydride können NaBH₄, LiBH₄, KBH₄, NaAlH₄, LiAlH₄, KAlH₄, Mg(BH₄)₂, Ca(BH₄)₂, Mg(AlH₄)₂, Ca(AlH₄)₂ verwendet werden. Komplexe Metallhydride der Bor- oder Aluminiumgruppe können die allgemeine Formel M¹ M² HₙLₘ haben, worin n eine ganze Zahl von 1 bis 4 ist und m eine ganze Zahl von 4 bis 4 minus der entsprechenden Zahl n ist, M¹ ein Alkalimetallatom bedeutet, M² Bor oder Aluminium bedeutet und L C₁₋₄-Alkyl, C₁₋₄-Alkenyl, C₁₋₄-Alkoxy, CN oder ein Amin ist oder die komplexen Metallhydride können die allgemeine Formel M²H_{O}Lₚ haben, worin M² die genannte Bedeutung hat und O eine ganze Zahl von 0 bis 3 ist und p eine ganze Zahl von 3 bis 3 minus der entsprechenden Zahl p ist. Als M¹ M² HₙLₘ können LiBH (C₂H₅)₃, LiBHₓ (OCH₃)₄₋ₓ, worin x eine ganze Zahl von 1 bis 3 bedeutet, LiAlH(OC(CH₃)₃), NaAlH₂(OC₂H₄OCH₃)₂, NaAlH₂(C₂H₅)₂ oder NaBH₃CN eingesetzt werden. Vorzugsweise wird die Reduktion mit einem Metallborhydrid durchgeführt.
Wie fachmännisch bekannt, können die erwähnten Metallhydride wie z. B. LiBH₄ auch "in situ" erzeugt werden. Gängige Darstellungsmethoden für LiBH₄ sind beispielsweise die Umsetzung eines Alkalimetallborhydrids mit einem Lithiumhalogenid (H. C. Brown et al., Inorg. Chem. 20, 1981, 4456 - 4457), die Umsetzung von LiH mit B₂O₃ in Gegenwart von Wasserstoff und einem Hydrierkatalysator (EP-A 0 512 895), die Umsetzung von LiH mit (H₅C₂)OBF₃ (DE-OS 94 77 02) und die von LiH mit B(OCH₃)₃ (US-A 2,534,533).

Zweckmässig werden die Metallhydride in einem molaren Verhältnis von 1 bis 5 pro mol (±)-2-Azabicyclo[2.2.1]hept-5-en-3-on eingesetzt.

Vorzugsweise werden die Metallhydride, insbesondere NaBH₄, mit Lithiumsalzzusätzen verwendet. Als Lithiumsalze können LiCl, LiBr, LiF, LiJ, Li₂SO₄, LiHSO₄, Li₂CO₃, Li(OCH₃) und LiCO₃ verwendet werden.

Zweckmässig wird die Reduktion unter Inertgasatmosphäre, beispielsweise unter Argon- oder Stickstoffatmosphäre, durchgeführt.

Die Reduktion kann bei einer Temperatur von -20 bis 200 °C, vorzugsweise bei einer Temperatur von 60 bis 150 °C, durchgeführt werden.

Als Lösungsmittel sind aprotische oder protische organische Lösungsmittel geeignet. Als aprotische organische Lösungsmitten können Ether oder Glykolether wie beispielsweise Diethylether, Dibutylether, Ethylmethylether, Diisopropylether, tert-Butylmethylether, Anisol, Dioxan, Tetrahydrofuran, Mono-Glyme, Diglyme und Formaldehyddimethylacetal eingesetzt werden. Alsprotische organische Lösungsmitten sind C₁₋₆-Alkohole wie Methanol, Ethanol, Propanol, Isopropanol, Butanol, tert-Butanol, Pentanol, tert-Amylalkohol oder Hexanol sowie Mischungen von diesen mit Wasser geeignet. Als protische organische Lösungsmittel ebenso geeignet sind Mischungen von einem der genannten Ether, Glykolether mit Wasser oder mit einem der genannten Alkohole wie eine Mischung von einem-C₁₋₆-Alkohol mit einem Ether oder Glykolether, insbesondere eine Mischung von Methanol, Ethanol oder Wasser mit Diethylether, Tetrahydrofuran, Dioxan, Glyme oder Diglyme. Vorzugsweise wird als Lösungsmittel ein protisches organisches Lösungsmittel, wie eine Mischung von einem C₁₋₆₋Alkohol oder Wasser mit einem Ether oder Glykolether, eingesetzt.

In einer bevorzugten Ausführungsform wird die Reduktion in Gegenwart eines Zusatzes, beispielsweise in Gegenwart von Wasser oder eines ein- oder mehrwertigen C₁₋₆-Alkohols durchgeführt. Als einwertige C₁₋₆-Alkohol können Methanol, Ethanol, Methoxyethanol, n-Propanol, Isopropanol, Isobutanol, tert-Butanol, n-Butanol verwendet werden. Als mehrwertige Alkohole können bspw. Diole wie Butandiol und Triole wie Glycerin verwendet werden. Insbesondere wird als C₁₋₆-Alkohol Methanol oder Ethanol verwendet. Zweckmässig wird dabei der C₁₋₆-Alkohol in einem molaren Verhältnis von 2 bis 15 pro mol (±)-2-Azabicyclo[2.2.1]hept-5-en-3-on eingesetzt.

Wird die Umsetzung in Gegenwart der genannten Alkohole durchgeführt, kann in situ (intermediär) der entsprechende Aminosäureester gebildet werden. D. h. wird als Edukt (+)-2-Azabicyclo[2.2.1]hept-5-en-3-on verwendet, kann erfindungsgemäss der entsprechende (+)-Aminosäureester gebildet werden.

Wird als Edukt (-)-2-Azabicyclo[2.2.1]hept-5-en-3-on verwendet, kann erfindungsgemäss dementsprechend der (-)-Aminosäureester intermediär gebildet werden.

Die Acylierung kann mit einem Carbonsäurehalogenid der allgemeinen Formel 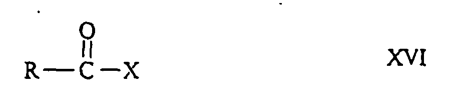 worin X ein Halogenatom bedeutet und R die genannte Bedeutung hat, oder mit einem Carbonsäureanhydrid der allgemeinen Formel 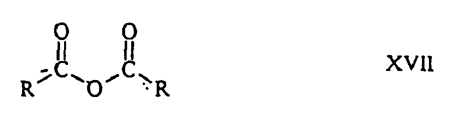 worin R die genannte Bedeutung hat, durchgeführtwerden.
Als Halogenatom X können F, Cl, Br oder I verwendet werden. Vorzugsweise wird Cl oder F verwendet.

Beispiele für Carbonsäurehalogenide sind: Acetylchlorid, Chloracetylchlorid, Buttersäurechlorid, Isobuttersäurechlorid, Phenylessigsäurechlorid, Chlorameisensäurebenzylester (Cbz-Cl), Propionsäurechlorid, Benzoylchlorid, Chlorameisensäurealkylester oder tert-Butyloxycarbonylfluorid.

Beispiele für Carbonsäureanhydride sind tert-Butoxycarbonylanhydrid, Buttersäureanhydrid, Essigsäureanhydrid oder Propionsäureanhydrid. Vorzugsweise wird die Acylierung mit einem Carbonsäureanhydrid, insbesondere mit tert-Butoxycarbonylanliydrid durchgeführt.
Die Acylierung kann ohne Lösungsmittel oder mit einem aprotischen organischen Lösungsmittel durchgeführt werden. Zweckmässig wird die Acylierung in einem aprotischen organischen Lösungsmittel durchgeführt. Als aprotisches organisches Lösungsmittel sind beispielsweise Pyridin, Acetonitril, Dimethylformamid, Diisopropylether, Tetrahydrofuran, Toluol, Methylenchlorid, N-Methylpyrrolidon, Triethylamin, Chloroform, Essigsäureethylester und Essigsäureanhydrid bzw. Mischungen von diesen geeignet.

Zweckmässig wird die Acylierung bei einer Temperatur von -20 bis 100 °C, vorzugsweise von 0 bis 80 °C, durchgeführt.

Die erfindungsgemässe Weiterumsetzung vom (1R,4S)- oder (1S,4R)-1-Amino-4-(hydroxymethyl)-2-cyclopentenhydrogen-D- oder L-tartrat zum (1S,4R)- oder (1 R,4S)-4-(2-amino-6-chlor-9-H-purin-9-yl)-2-cyclopenten-1-methanol, oder dessen Salzen, der Formeln 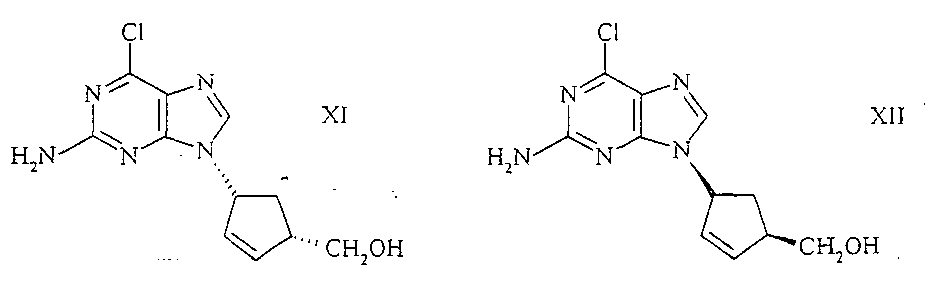 wird derart durchgeführt, dass man (1R,4S)- oder (1S,4R)-1-Amino-4-(hydroxymethyl)-2-cyclopentenhydrogen-D- oder L-tartrat mit N-(2-amino-4,6-dichlorpyrimidin)-5-yl)formamid der Formel 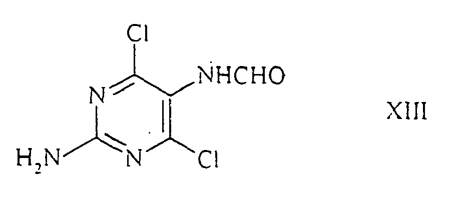 ins (1S,4R)- oder (1R,4S)-4-[(2-amino-6-chlor-5-formamido-4-pyrimidinyl)-amino]-2-cyclopenien-1-methanol der Formeln 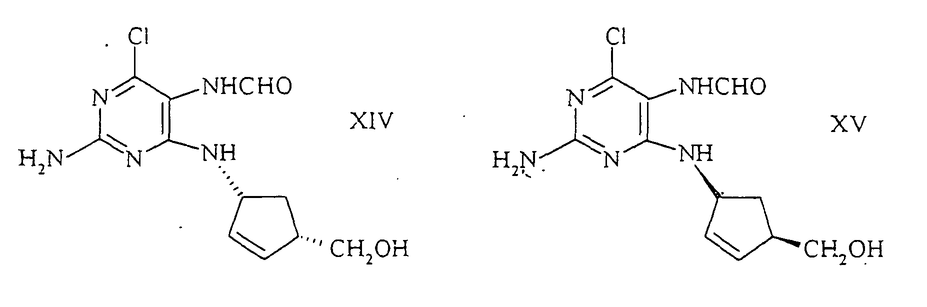 überführt und letzteres dann auf bekannte Weise zu den Verbindungen gemäss Formel XI und XII cyclisiert.

Das N-(2-amino-4,6-dichlorpyrimidin)-5-yl)formamid kann gemäss der WO 95 / 21 161 hergestellt werden.

Zweckmässig wird die Umsetzung in Gegenwart einer Base durchgeführt. Als Basen sind die gleichen geeignet, die wie zuvor beschrieben zur Freisetzung der (1R,4S)- oder (1S,4R)-1-Amino-4-(hydroxymethyl)-2-cyclopentene aus dem entsprechenden Tartrat eingesetzt werden.

Zweckmässig wird die Umsetzung in einem protischen Lösungsmittel durchgeführt. Als protisches Lösungsmittel können C₁₋₆-Alkohole wie Methanol, Ethanol, Propanol, Isopropanol, Butanol oder Isobutanol verwendet werden.

Dann wird das (1S,4R)- oder (1R,4S)-4-[(2-amino-6-chlor-5-formamido-4-pyrimidinyl)-amino]-2-cyclopenten-1-methanol der Formel XIV oder XV auf bekannte Weise gemäss der WO 95 / 21 161 ins Endprodukt gemäss Formel XI oder XII cyclisiert.

Üblicherweise wird die Cyclisierung gelöst in Trialkylorthoformiaten in Gegenwart von einer konzentrierten wässrigen Säure durchgeführt. Als Trialkylorthoformiate können beispielsweise Trimethyl- oder Triethylorthoformiat Verwendung finden.
Als wässrige Säure kann beispielsweise Chlorwasserstoffsäure, Schwefelsäure oder Methansulfonsäure verwendet werden.

### Beispiele:

### Beispiel 1

### Reduktion von -Azabicyclo[2.2.1]hept-5-en-3-on

### 1.1 Herstellung von cis-(±)-1-Amino-4-(hydroxymethyl)-2-cyclopenten

Eine Suspension von (±)-2-Azabicyclo[2.2.1]hept-5-en-3-on (10,00 g, 91,6 mmol) und Lithiumborhydrid (4,00 g, 183,7 mmol) in trockenem Dioxan (100 ml) wurde unter Inertgasatmosphäre (Argon) 4 h bei 110°C unter Rückflusstemperatur erhitzt. Nach dieser Zeit waren ca. 20 - 25 % des Eduktes zum Produkt umgesetzt (GC-Analyse mit internem Standard Benzophenon nach Aufarbeitung des Reaktionsgemisches; Aufarbeitung: 0,05 ml des Reaktionsgemisches wurden mit 0,1 ml 1M HCl gequencht und direkt im Anschluss mit 0,2 ml 1M NaOH basisch gestellt).

Der Strukturnachweis des Produktes erfolgte durch H-NMR, GC und GC-MS.

### 1.2 Herstellung von cis-(+)-1-Amino-4-(hydroxymethyl)-2-cyclopenten

In einem 25 ml Rundkolben wurden 1,0 g (9,2 mmol) (÷)-2-Azabicyclo[2.2.1]hept-5-en-3-on und 0,4 g (18,4 mmol) Lithiumborhydrid, unter Inertgasatmosphäre, in 10 ml Dioxan vorgelegt und 3 h bei 110 °C refluxiert. Überschüssiges Reduktionsmittel wurde durch Zugabe von ca. 5 ml halbkonzentrierter HCl vernichtet (auf pH 3 gestellt). Sofort im Anschluss daran wurde durch Zugabe von ca. 1 ml gesättigter NaHCO₃₋Lösung bei pH 8 gepuffert. GC Analyse zeigte hier die Bildung des Produktes. Das gesamte Reaktionsgemisch wurde nun zur Trockene eingedampft und mittels Säulenchromatographie (Gradient: Hexan/Essigester/MeOH = 1:1:1 -> MeOH) gereinigt. Auf diese Weise wurde cis-(+)-2-Azabicyclo[2.2.1]hept-5-en-3-on reisoliert und der entsprechende (+)-Aminoalkohol gewonnen.

### 1.3 Herstellung von cis-(-)-1-Amino-4-(hydroxymethyl)-2-cyclopenten

In einem 25 ml Rundkolben wurden 1,0 g (9,2 mmol) (-)-2-Azabicyclo[2.2.1]hept-5-en-3-on und 0,4 g (18,4 mmol) Lithiumborhydrid, unter Inertgasatmosphäre, in 10 ml Dioxan vorgelegt und 3 h bei 110°C refluxiert. Überschüssiges Reduktionsmittel wurde durch Zugabe von ca. 5 ml halbkonzentrierter HCl vernichtet (auf pH 3 gestellt). Sofort im Anschluss daran wurde durch Zugabe von ca. 1 ml gesättigter NaHCO₃₋Lösung bei pH 8 gepufferet. GC Analyse zeigte hier die Bildung des Produktes in 18 % Ausbeute an. (GC-Standard ist Benzophenon). Das gesamte Reaktionsgemisch wurde nun zur Trockene eingedampft und mittels Säulenchromatographie (Gradient: Hexan/Essigester/MeOH = 1:1:1 -> MeOH) gereinigt. Auf diese Weise wurde 0,43g (43 %) cis-(-)-2-Azabicyclo[2.2.1]hept-5-en-3-on reisoliert sowie 0,04g (4 %) entsprechender (-)-Aminoalkohol gewonnen. Durch HPLC war nur das (-)-Enantiomer des Aminoalkohols detektierbar. Der ee des Produktes ist somit >98 %.

### 1.4 Herstellung von cis-(±)-1-Amino-4-(hydroxymethyl)-2-cyclopenten in einem Alkohol

In einem 100 ml Rundkolben mit magnetischer Rührung wurden 3,0 g (27,5 mmol) (±)-2-Azabicyclo[2.2. 1]hept-5-en-3-on und 1,2 g (28,3 mmol) Lithiumborhydrid, unter Inertgasatmosphäre, in 35 g 2-Butanol vorgelegt und 3 h bei 60 °C gerührt. GC-Analyse eines Musters (Aufarbeitung: 0,1 g Probe mit 0,2 ml lM HCl sauergestellt, dann mit 0,1 ml gesättigter NaHCO₃ rasch basisch gestellt) zeigte nach dieser Zeit die Bildung des Produktes in 12 %iger Ausbeute an (GC-Standard ist Benzophenon).

### 1.5 Herstellung von cis-(±)-1-Amino-4-(hydroxymethyl)-2-cyclopenten in einem Alkohol/Ether-Gemisch

In einem 10 ml Rundkolben wurden unter Inertgasatmosphäre 0,5 g (4,6 mmol) (±)-2-Azabicyclo[2.2.1]hept-5-en-3-on und 0,59 g (18,4 mmol) Methanol in 7,5 ml Dioxan (abs.) vorgelegt Dazu gab man 0,21 g (9,2 mmol) Lithiumborhydrid und heizte für 4 h bei 60°C. Dann kühlte man mit einem Eis/Wasser Bad auf 5°C und gab vorsichtig ca. 10 ml halbkonzentrierte HCl zum Reaktionsgemisch (heftige Reaktion, Gasentwicklung), wodurch eine gelblich klare Lösung entstand. Diese Lösung wurde direkt durch ein quantitatives ionenchromatografisches Verfahren analysiert Sie enthielt 0,60 mmol (13,1%) cis-(±)-2-Azabicyclo[2.2.1]hept-5-en-3-on (bestimmt als HCl-Salz der entsprechenden Aminosäure, welche das saure Hydrolyseprodukt des (±)-2-Azabicyclo[2.2.1]hept-5-en-3-ons ist) und 3,06 mmol Produkt, entsprechend einer Ausbeute von 66,8%, Aminoalkohol.

### 1.6 Herstellung von cis-(±)-1-Amino-4-(hydroxymethyl)-2-cyclopenten in Gegenwart von Zusätzen wie Wasser oder verschiedenen Alkoholen

In einem 10 ml Rundkolben wurden 0,50 g (4,6 mmol) (±)-2-Azabicyclo[2.2.1]hept-5-en-3-on und 0,30 g (13,7 mmol) Lithiumborhydrid in 7,5 ml abs. Dioxan vorgelegt und auf 60 °C aufgeheizt. Bei dieser Temperatur wurde während 30 min. mittels Spritze X mmol des Zusatzes Y (Alkohol oder Wasser) zugetropft. Nun wurde 2 h bei 60 °C gerührt, abgekühlt auf ca. 20 °C und auf ca. 10 ml halbkonzentrierte HCl gegeben. Der Gehalt wurde dann direkt durch ein quantitatives ionenchromatografisches Verfahren bestimmt (vgl. Tabelle 1).

**Tabelle 1**

| Beispiel | Zusatz Y | X | X | (±)-2-Azabi- cyclo[2.2.1]-hept-5-en-3-on | Aminoalkohol |
|---|---|---|---|---|---|
| | | mmol | Aequivalente | Ausbeute % | |
| 1.6.1 | - | - | - | 15 | 52 |
| 1.6.2 | Wasser | 17,1 | 1,25 | 23,3 | 67,5 |
| 1.6.3 | Wasser | 34,3 | 2,5 | 32,3 | 58,3 |
| 1.6.4 | Methanol | 34,3 | 2,5 | 4,5 | 83,1 |
| 1.6.5 | Ethanol | 34,3 | 2,5 | 6,5 | 74,7 |
| 1.6.6 | Isopropanol | 34,3 | 2,5 | 28,1 | 52,3 |

### 1.7 Herstellung von cis-(±)-1-Amino-4-(hydroxymethyl)-2-cyclopenten mit verschiedenen Methanol-Mengen

Die Reaktion wurde unter den gleichen Bedingungen wie Beispiel 1.6 durchgeführt, jedoch mit der Ausnahme, dass anstatt des Zusatzes Y die Reaktion in verschiedenen Methanol-Konzentrationen durchgeführt wurde. Die Ergebnisse sind in Tabelle 2 zusammengefasst.

**Tabelle 2**

| Beispiel | Methanol | Methanol | (±)-2-Azabicyclo-[2.2.1] hept-5-en-3-on | Aminoalkohol |
|---|---|---|---|---|
| | mmol | Aequivalente | Ausbeute % | |
| 1.7.1 | 9,2 | 1 | 27,5 | 44,8 |
| 1.7.2 | 18,3 | 2 | 13,1 | 66,8 |
| 1.7.3 | 27,5 | 3 | 24,7 | 54,8 |
| 1.7.4 | 36,6 | 4 | 5,7 | 56,8 |
| 1.7.5 | 45,8 | 5 | 12,0 | 58,3 |
| 1.7.6 | 55,0 | 6 | 7,2 | 33,0 |

### 1.8 Herstellung von cis-(±)-1-Amino-4-(hydroxymethyl)-2-cyclopenten mit verschiedenen Lösungsmitteln

Die Reaktion wurde unter den gleichen Bedingungen wie Beispiel 1.6 durchgeführt, jedoch mit der Ausnahme, dass anstatt des Zusatzes Y 1,1 g Methanol zugetropft wurden und anstatt Dioxan als Lösungsmittel die Reaktion in verschiedenen Lösungsmitteln (7,5 ml) durchgeführt wurde und der Gehalt bestimmt wurde. Die Ergebnisse sind in Tabelle 3 zusammengefasst.

**Tabelle 3**

| Beispiel | Lösungsmittel X | (±)-2-Azabicyclo[2.2.1] hept-5-en-3-on | Aminoalkohol |
|---|---|---|---|
| | | Ausbeute % | |
| 1.8.1 | Dioxan | 13,6 | 79,8 |
| 1.8.2 | Diethylether | 10,8 | 68,6 |
| 1.8.3 | Tetrahydrofuran | 22,4 | 67,6 |
| 1.8.4 | Diisopropyl-Ether | 12,6 | 51,3 |
| 1.8.5 | tert-Butylmethyl-Ether | 10,0 | 71,3 |
| 1.8.6 | Mono-Glyme | 15,5 | 75,3 |
| 1.8.7 | Formaldehyddimethylacetal | 12,0 | 74,2 |

### 1.9 Herstellung von cis-(±)-1-Amino-4-(hydroxymethyl)-2-cyclopenten mit verschiedenen LiBH₄-Zugaben

Die Reaktion wurde unter den gleichen Bedingungen wie in Beispiel 1.6 durchgeführt, jedoch mit der Ausnahme, dass anstatt Zusatz Y 2,5 mol Methanol eingesetzt wurden und die Reaktion mit verschiedenen LiBH₄-Konzentrationen durchgeführt und der Gehalt bestimmt wurde. Die Ergebnisse sind in Tabelle 4 zusammengefasst.

**Tabelle 4**

| Beispiel | LiBH₄ | LiBH₄ | (±)-2-Azabicyclo[2.2.1] hept-5-en-3-on | Aminoalkohol |
|---|---|---|---|---|
| | mmol | Aequivalente | Ausbeute % | |
| 1.9.1 | 4,6 | 1 | 11,9 | 47,9 |
| 1.9.2 | 6,9 | 1,5 | 9,6 | 45,6 |
| 1.9.3 | 9,2 | 2 | 12,7 | 71,3 |
| 1.9.4 | 11,5 | 2,5 | 13,3 | 74,5 |
| 1.9.5 | 13,8 | 3 | 12,8 | 77,1 |
| 1.96 | 16,1 | 3,5 | 12,7 | 62,4 |

### 1.11 Herstellung von cis-(+)- oder (-)-1-Amino-4-(hydroxymethyl)-2-cyclopenten in Gegenwart verschiedener Akohole bzw. in Gegenwart von Wasser in verschiedenen Lösungsmitteln

In einem 10 ml Rundkolben mit magnetischer Rührung wurden 0,50 g (4,6 mmol) (+)- oder (-)-3-Azabicyclo[2.2.1]hept-5-en-3-on und 0,30 g (13,7 mmol) Lithiumborhydrid in 6 ml verschiedener Lösungsmittel vorgelegt und auf 60°C aufgeheizt. Bei dieser Temperatur wird während 30 min. mittels Spritze 34,3 mmol des Zusatzes Y zugetropft. Nun wurde 2h bei 60°C gerührt, abgekühlt auf ca. 20°C und auf ca. 10 ml halbkonzentrierte HCl gegeben.
Der Gehalt wird dann direkt durch ein quantitatives ionenchromatografisches Verfahren bestimmt (vgl. Tabelle 5). Der ee-Wert des Produktes wurde mittels HPLC ermittelt.
Die Ergebnisse sind in Tabelle 5 zusammengefasst.

**Tabelle 5**

| | (-)-2-Azabicyclo[2.2.1] hept-5-en-3-on | (+)-2-Azabicyclo-[2.2.1]hept-5-en-3-on | | | Aminoalkohol | |
|---|---|---|---|---|---|---|
| Beispiel | ee-Wert | | Lösungsmittel | Zusatz Y | Ausbeute (IC) | ee-Wert (HPLC) |
| 1.1.0.1 | 98,0 | | Dioxan | Wasser | 64,3 | >99,0 |
| 1.10.2 | 98,0 | | Glyme | Wasser | 68,0 | >99,0 |
| 1.10.3 | 75,9 | | Dioxan | Wasser | 65,1 | 76,0 |
| 1.10.4 | 75,9 | | Glyme | Wasser | 63,5 | 75,6 |
| 1.10.5 | 50,2 | | Dioxan | Wasser | 74,8 | 51,4 |
| 1.10.6 | 51,6. | | Glyme | Wasser | 64,1 | 53,0 |
| 1.10.7 | 25,3 | | Dioxan | Wasser | 61,1 | 30,4 |
| 1.10.8 | 25,6 | | Glyme | Wasser | 61,0 | 29,6 |
| 1.10.9 | 98,0 | | Dioxan | Methanol | 83,1 | 98,2 |
| 1.10.10 | 98,0 | | Glyme | Methanol | 81,5 | 99,2 |
| 1.10.11 | 75,9 | | Dioxan | Methanol | 81,4 | 78,0 |
| 1.10.12 | 76,2 | | Glyme | Methanol | 79,9 | 78,6 |
| 1.10.13 | 50,4 | | Dioxan | Methanol | 81,3 | 54,4 |
| 1.10.14 | 51,5 | | Glyme | Methanol | 82,0 | 55,2 |
| 1.10.15 | 24,8 | | Dioxan | Methanol | 65,2 | 27,4 |
| 1.10.16 | 27.8 | | Glyme | Methanol | 81,7 | 32.2 |
| 1.10.17 | 98,0 | | Dioxan | Ethanol | 80,8 | 80,8 |
| 1.10.18 | 98,0 | | Glyme | Ethanol | 85,1 | 85,1 |
| 1.10.19 | 75,5 | | Dioxan | Ethanol | 85,3 | 78,2 |
| 1.10.20 | 75,6 | | Glyme | Ethanol | 83,6 | 78,4 |
| 1.10.21 | 50,7 | | Dioxan | Ethanol | 76,3 | 54,4 |
| 1.10.22 | 51,1 | | Glyme | Ethanol | 71,3 | 55,2 |
| 1.10.23 | 25,4 | | Dioxan | Ethanol | 73,0 | 28,6 |
| 1.10.24 | 25,5 | | Glyme | Ethanol | 7.5,0 | 28,6 |
| 1.10.25 | | 98,0 | Dioxan | Wasser | 62,0 | >99,0 |
| 1.10.26 | | 98,0 | Glyme | Wasser | 59,5 | >99,0 |
| 1.10.27 | | 51,3 | Dioxan | Wasser | 79,0 | 52,2 |
| 1.10.28 | | 49,0 | Glyme | Wasser | 61,3 | 52,0 |
| 1.10.29 | | 98,0 | Dioxan | Methanol | 77,2 | >99,0 |
| 1.10.30 | | 98,0 | Glyme | Methanol | 80,0 | >99,0 |
| 1.10.31 | | 49,0 | Dioxan | Methanol | 80,8 | 46,8 |
| 1.10.32 | | 49,5 | Glyme | Methanol | 80,9 | 48,8 |

### 1.11 Herstellung von cis-(±)-1-Amino-4-(hydroxymethyl)-2-cyclopenten mittels Natriumborhydrid in verschiedenen Alkoholen

Entsprechend zu Beispiel 1.6 wurde die Reaktion in verschiedenen Zusätzen (Alkohole oder Wasser) durchgeführt. Im Unterschied zu Beispiel 1.6 wurde jedoch Natriumborhydrid (0,51g; 13,7 mmol) als Reduktionsmittel eingesetzt. Die Ergebnisse sind in Tabelle 6 zusammengefasst.

**Tabelle 6**

| Beispiel | Zusatz Y | X | X | (±)-2-Azabicyclo[2.2.1] hept-5-en-3-on | Aminoalkohol |
|---|---|---|---|---|---|
| | | mmol | Aequivalente | Ausbeute % | |
| 1.11.1 | Wasser | 17,1 | 1,25 | 75,4 | 20,1 |
| 1.11.2 | Wasser | 34,3 | 2,5 | 71,9 | 26,7 |
| 1.11.3 | Methanol | 34,3 | 2,5 | 39,2 | 22,2 |
| 1.11.4 | Ethanol | 34,3 | 2,5 | 67,8 | 8,6 |
| 1.11.5 | - | - | - | 62,2 | 3,5 |

### 1.13 Herstellung von cis-(±)-1-Amino-4-(hydroxymethyl)-2-cyclopenten mit NaBH₃CN

In einem 100 ml Sulfierkolben mit mechanischer Rührung wurden 60 ml Dioxan und 8,6 g (137 mmol) Natriumcyanborhydrid sowie 11,9 g (137 mmol) Lithiumbromid über Nacht für 15 h bei 110°C rückflussiert. Dann wurde auf 60°C abgekühlt und eine Lösung von 5,0 g (45,8 mmol) (±)-2-Azabicyclo[2.2.1]hept-5-en-3-on mit 15ml Methanol während 30 min. zugetropft. Die weisse Suspension wurde für 3 h bei 60°C gerührt, abgekühlt auf ca. 5°C.und auf ca. 100 ml halbkonzentrierte HCl gegeben. Der Gehalt wurde dann direkt durch ein quantitatives ionenchromatografisches Verfahren bestimmt. Die Ausbeute an Aminoalkohol betrug ca. 4%.

### Beispiel 2: Herstellung von (1R,4S)-oder (1S,4R)-1-Amino-4-(hydroxymethyl)-2-cyclopenten

### 2.1 Racematspaltung mit D-(-)-Weinsäure

2.1.1 Ein Gemisch aus 8 g (70,6 mmol) racemisches 1-Amino-4-(hydroxymethyl)-2-cyclopenten und 10,6 g (70,6 mmol) D-(-)-Weinsäure in 186 g Methanol wurde bei Rückflusstemperatur gelöst. Anschliessend wurde in 2 h auf 20 °C gekühlt. Bei 43 °C wurden Impfkristalle des reinen (1R,4S)-1-Amino-4-(hydroxymethyl)-2-cyclopenten hydrogen-D-tartrat zugegeben. Das kristallisierte Produkt wurde abfiltriert und getrocknet. Ausbeute : 8,49 g (45,6 % bezogen auf racemisches Edukt) (1R,4S)-1-Amino-4-(hydroxymethyl)-2-cyclopenten hydrogen-D-tartrat, ee-Wert : 91,1 %. Zur Reinigung wurden 8,49 g (32,25 mmol)⁻des Hydrogentartrats in 30 ml Methanol suspendiert und 2 Äquivalente 30 % Natriummethylat zugegeben. Das Natriumtartrat wurde abfiltiert und das Methanol abdestilliert.
Der Rückstand würde in 35 ml Pentanol aufgenommen. Darauf wurden bei 55 °C 1,5 g HCl eingeleitet, die Lösung wurde langsam abgekühlt. Bei 40°C wurde die Lösung mit (1R,4S)-1-Amino-4-(hydroxymethyl)-2-cyclopenten-hydrochlorid angeimpft. Anschliessend wurden 45 ml Aceton zudosiert, die Suspension langsam auf 0 °C abgekühlt, filtriert und der Rückstand getrocknet.
Es wurden 3,91 g (1R,4S)-1-Amino-4-(hydroxymethyl)-2-cyclopenten hydrochlorid mit einem ee-Wert von > 98 % erhalten, entsprechend einer Ausbeute bezogen auf eingesetztes racemisches (1R,4S)-1-Amino-4-(hydroxymethyl)-2-cyclopenten von 37 %.

2.1.2 Ein Gemisch aus 64 g racemisches 1-Amino-4-(hydroxymethyl)-2-cyclopenten (0,5 mol) und 75,2 g D-(-)-Weinsäure in 1330 g Methanol wurde bei Rückflusstemperatur gelöst und anschliessend in 2 h auf 20°C gekühlt. Bei 43 °C wurden Impfkristalle des reinen. 1R,4S-Enantiomers zugegeben. Das kristallisierte Produkt wurde abfiltriert und getrocknet. Ausbeute : 63,2 g (48,0 % bezogen auf racemisches 1-Amino-4-(hydroxymethyl)-2-cyclopenten) 1R,4S-(1-Amino-4-(hydroxymethyl)-2-cyclopenten-hydrogentartrat, ee-Wert : 91,1 %. Der ee-Wert in der Mutterlauge betrug 76,0 %.

### 2.1.3 Umkristallisation von 1R,4S-(4-Amino-2-cyclopenten-1-yl)-methanol-D-hydrogentartrat

61,84 g 1R,4S-(4-Amino-2-cyclopenten-1-yl)methanol-D-hydrogentartrat (0,235 mol, ee-Wert 91,1 %) wurden gelöst in 752 g Methanol unter Rückfluss. Die Lösung wurde abgekühlt auf 20 °C innert 90 min., anschliessend wurde das Produkt abfiltriert und mit 64 g kaltem Methanol nachgewaschen. Nach Trocknung wurde 54,56 g 1R,4S-(4-Amino-2-cyclopenten-1-yl)methanol-D-hydrogentartrat erhalten, ee-Wert 99,4 % (Ausbeute 88,2 %, 42,3 % bezüglich racemischem 1-Amino-4-(hydroxymethyl)-2-cyclopenten). Dieses wurde tel quel in die Chlorpurinsynthese eingesetzt.

2.1.4 Analog zu Beispiel 2.1.2, aber mit 223 g Methanol und Animpfen bei 50 °C wurde die Racemattrennung durchgeführt. Die Ausbeute betrug 7,98 g (42,9 % bezogen auf eingesetztes racemisches (11R,4S)-1-Amino-4-(hydroxymethyl)-2-cyclopenten).

### 2.2 Racematspaltung mit L-(+)-Weinsäure

2.2.1 Ein Gemisch aus S g (70,6 mmol) racemischem 1-Amino-4-(hydroxymethyl)-2-cyclopenten und 10,6 g (70,6 mmol) L-(+)-Weinsäure in 186 g Methanol wurde bei Rückflusstemperatur gelöst Anschliessend wurde in 2 h auf 20 °C gekühlt. Bei 43 °C wurden Impfkristalle des reinen (1S,4R)-1-Amino-4-(hydroxymethyl)-2-cyclopenten hydrogen-L-tartrat zugegeben. Das kristallisierte (1S,4R)-1-Amino-4-(hydroxymethyl)-2-cyclopenten hydrogen-L-tartrat wurde abfiltriert und getrocknet. (ee-Wert : 91,1 %) Zu der Mutterlauge wurden 14 g 30 % methanolisches Natriummethylat zugegeben, darauf das Methanol eingeengt. Der Rückstand wurde in 35 ml Isobutanol aufgenommen und das unlösliche Natriumtartrat abfiltriert. In das Filtrat wurden bei 55 °C 2 g gasförmige HCl eingeleitet. Man gab dann 38 ml Aceton hinzu und liess innerhalb von 1 h auf 10 °C abkühlen. Nach 1 h wurde das (1R,4S)-1-Amino-4-(hydroxymethyl)-2-cyclopenten-hydrochlorid abgenutscht und mit 8 ml Aceton nachgewaschen. Nach dem Trocknen im Vakuum erhielt man das (1R,4S)-1-Amino-4-(hydroxymethyl)-2-cyclopenten-hydrochlorid in einer Ausbeute von 34 g 31,6 % bezogen auf racemisches 1-Amino-4-(hydroxymethyl)-2-cyclopenten mit einem ee-Wert von > 98 %.

### Beispiel 3: Herstellung von (1R,4S)-Amino-4-(hydroxymethyl)-2-cyclopentenhydrochlorid

### 3.1 Reduktion von (-)-2-Azabicyclo[2.2.1]hept-5-en-3-on

In einen mit N₂ inertisierten 2 l- Autoklaven (V4A) wurden 61,4 g Natriumborhydrid 97,5% (1,623 Mol), 70,2 g Lithiumchlorid 98,5% (1,656 Mol), 13,2 g Celite sowie 1410 g Tetrahydrofuran eingefüllt. Der Autoklav wurde verschlossen, es wurde auf eine Innentemperatur von 130 °C aufgeheizt und 4,5 Stunden bei dieser Temperatur gerührt (max. 8,0 bar). Nach Abkühlen auf ca. 60 °C wurden die in Tetrahydrofuran unlöslichen Natriumsalze (NaCl, NaBH₄) abfiltriert. Diese wurden mit 353 g Tetrahydrofuran gewaschen und die vereinigten Filtrate wurden in einem 1 1-Glasrührwerk durch Destillation bei Normaldruck auf ca. die Hälfte eingeengt (Destillat 1: ca. 710 g Tetrahydrofuran). Anschliessend wurde durch weitere Destillation, im Wechsel mit portionsweiser Zugabe von insgesamt 936 g Dioxan der Lösungsmitteltausch vollendet (Destiilat 2: ca. 1289 g Tetrahydrofuran/Dioxan).
Zur auf ca. 60 °C gekühlten LiBH₄-Suspension wurden 56,7 g (-)-2-Azabicyclo[2.2.1]hept-5-en-3-on (97,5%) addiert.
Bei ca. 60 °C beginnend wurden in exakt einer Stunde 132,5 g Methanol so zudosiert, dass ein Temperaturbereich von 58 - 62 °C eingehalten wurde. Es wurde eine weitere Stunde bei 60 °C nachreagieren gelassen. Anschliessend wurden weitere 397,0 g Methanol addiert (Probe enthält eine analytische Ausbeute von 70,5%) und der Rührwerksinhalt wurde auf 0 °C abgekühlt. Bei dieser Temperatur wurden 90,0 g HCl in die Reaktionsmischung eingeleitet (leicht exotherm) und es wurde eine weitere Stunde bei ca 0 °C gerührt. Durch Destillation bei Normaldruck (bis zu einer Kopftemperatur von 75 °C) wurden die Leichtsieder (Methanol, Borester) und ca. 70% des Dioxans entfernt (Destillat 3: ca. 1093 g). Anschliessend wurde durch Vakuumdestillation (ca. 30 mbar), im Wechsel mit portionsweiser Zugabe von insgesamt 282 g 1-Pentanol der Lösungsmitteltausch vollendet (Destillat 4: ca. 240 g Dioxan / Pentanol). Nach Zugabe weiterer 302 g 1-Pentanol wurde 1 Stunde bei 50 °C gerührt und die ausgefallenen Salze, ca. 39 g Feuchtgewicht, wurden abfiltriert und mit 200 g 1-Pentanol gewaschen. Die vereinigten Filtrate wurden durch erneute Vakuumdestillation (ca. 20 mbar) eingeengt (Destillat 5: ca. 235 g 1-Pentanol). Dann wurden bei ca. 50 °C 236 g Aceton zudosiert und die Reaktionsmischung wurde mit einigen Kristallen (1R,4S)-Amino-4-(hydroxymethyl)-2-cyclopenten angeimpft, Innerhalb einer Stunde wurde auf 5 °C abgekühlt und zur Vollendung der Kristallisation wurde weitere 6 h bei 5 °C gerührt.
Das Kristallisat wurde abfiltriert, mit 63 g Aceton gewaschen und bei max. 50 °C im Vakuumtrockenschrank (10 mbar) getrocknet. Man erhielt 83,5 g Rohprodukt * (Gehalt: 56,5%).
Dies entsprach einer Ausbeute von 61,4 % bzgl. eingesetztem (-)-2-Azabicyclo-[2.2.1]hept-5-en-3-on.

### 3.2 Reduktion von (±)-2-Azabicyclo[2.2.1]hept-5-en-3-on

In einen mit N₂ inertisierten 2 1 - Autoklaven (V4A) wurden 41,56 g Natriumborhydrid 97,5% (1,071 Mol), 51,48 g Lithiumchlorid 98,5% (1,196 Mol), 9,30 g Celite sowie 955,0 g Tetrahydrofuran eingefüllt. Der Autoklav wurde verschlossen, es wurde auf eine Innentemperatur von 130 °C aufgeheizt und 6 Stüriden-bei dieser Temperatur gerührt (max. 6,3 bar).
Nach Abkühlen auf ca. 60 °C wurden die in Tetrahydrofuran unlöslichen Natriumsalze (NaCI, NaBH₄) abfiltriert. Diese wurden mit 239,0 g Tetrahydrofuran gewaschen und die vereinigten Filtrate wurden in einem ll-Glasrührwerk durch Destillation bei Normaldruck auf ca die Hälfte eingeengt (Destillat 1: ca. 590 g THF). Anschliessend wurde durch weitere Destillation, im Wechsel mit portionsweiser Zugabe von insgesamt 661,0 g Dioxan der Lösungsmitteltausch vollendet (Destillat 2: ca. 685 g Tetrahydrofuran/Dioxan). Zur auf ca. 60 °C gekühlten LiBH₄-Suspension wurden 36,0 g 2-Azabicyclo[2.2.1]hept-5-en-3-on (97,5%) addiert. Bei ca. 60 °C beginnend wurden in exakt einer Stunde 77,6 g Methanol so zudosiert, dass ein Temperaturbereich von 58 - 62 °C eingehalten wurde. Es wurde eine weitere Stunde bei 60 °C nachreagieren gelassen. Anschliessend wurden weitere 233,0 g Methanol addiert und der. Rührwerksinhatt wurde auf 0 °C abgekühlt. Bei dieser Temperatur wurden 52,9 g HCl in die Reaktionsmischung eingeleitet (leicht exotherm) und es wurde eine weitere Stunde bei ca 0 °C gerührt.
Durch Destillation bei Normaldruck (bis zu einer Kopftemperatur von 75 °C) wurden die Leichtsieder (Methanol, Borester) und ca. 70% des Dioxans entfernt (Destillat 3: ca. 700 g).

Anschliessend wurde durch Vakuumdestillation (ca. 30 mbar), im Wechsel mit portionsweiser Zugabe von insgesamt 169,4 g 1-Pentanol der Lösungsmitteltausch vollendet (Destillat 4: ca. 183 g Dioxan / Pentanol). Nach Zugabe weiterer 127,1 g 1-Pentanol wurde 1 Stunde bei 50 °C gerührt und die ausgefallenen Salze, ca.41 g Feuchtgewicht, wurden abfiltriert und mit 63,5 g 1-Pentanol gewaschen. Die vereinigten Filtrate wurden durch erneute Vakuumdestillation (ca. 20 mbar) eingeengt (Destillat 5: ca. 235 g 1-Pentanol). Dann wurden bei ca. 50 °C 238,0 g Aceton zudosiert und die Reaktionsmischung wurde mit einigen Kristallen Aminoalkohol-Hydrochloridsalz angeimpft. Innerhalb einer Stunde wurde auf 5 °C abgekühlt und zur Vollendung der Kristallisation wurde weitere 6 Stunden bei 5 °C gerührt.
Das Kristallisat wurde abfiltriert, mit 61,0 g Aceton gewaschen und bei max. 50 °C im Vakuumtrockenschrank (10 mbar) getrocknet. Man erhielt 50,0 g Rohprodukt (Gehalt: ca. 50% Aminoalkohol-Hydrochloridsalz). Dies entsprach einer Ausbeute von 52,0 % bzgl. eingesetztem 2-Azabicyclo[2.2.1]hept-5-en-3-on.

### Beispiel 4: Herstellung von acylierten Aminoalkoholen

### 4.1 Herstellung von (1R,4S)-N-BOC-1-amino-4-(hydroxymethyl)-2-cyclopenten (BOC = tert-Butoxycarbonyl)

75 g einer Lösung von (1R,4S)-1-Amino-4-hydroxymethyl-2-cyclopenten wurde mit 30 %igen NaOH auf pH S gestellt und mit 6 g NaHCO₃ versetzt. Das Gemisch wurde auf 52 °C erwärmt. Unter guter Rührung wurde dazu 60 ml Diisopropylether zugegeben und dann während 2 h eine Lösung von 11,12 g BOC-Anhydrid in 18,2 ml Diisopropylether zudosiert. Die Mischung wurde über Celite filtriert und die Phasen getrennt. Die Wasserphase wurde mit 65 ml Diisopropylether extrahiert. Die vereinigten organischen Phasen wurden mit 45 ml Wasser gewaschen, dann auf 37,5 g eingedampft und auf 50 °C erwärmt. Zu der Lösung wurde 31 ml n-Hexan zugetropft.
Nach langsamer Kühlung auf 0 °C (2 h), wurde die Titelverbindung filtriert, mit 12 ml n-Hexan/ Diisopropylether 1/1 gewaschen und getrocknet. Man erhielt 6,75 g Produkt. Die Ausbeute betrug 71 %

### 4.2 Herstellung von (1R,4S)-N-Acetyl-1-amino-4-(hydroxymethyl)-2-cyclopenten

25 g (1R,4S)-1-Amino-4-(hydroxymethyl)-2-cyclopenten-hydrochlorid wurden in 182 ml Essigsäureanhydrid gelöst und bei 0 °C mit einer Lösung von 18,25 g Triethylamin in 60 ml Essigsäureanhydrid versetzt. Die Mischung wurde bei 80 °C 3 h gerührt, dann auf Raumtemperatur abgekühlt. Das Triethylaminhydrochlorid wurde abfiltriert und mit 120 ml n-Hexan gewaschen. Das Filtrat wurde eingedampft. Der Rückstand wurde mit 300 ml Toluol versetzt und bei Raumtemperatur in Gegenwart von 5,2 g Aktivkohle und 13 g Celite 20 Min. gerührt. Nach Filtration und Waschen des Filterkuchens (3 mal 40 ml Toluol), wurde das Lösungsmittel vollständig eingeengt. Der Rückstand wurde mit 180 ml Methanol und 15,5 g K₂CO₃ versetzt und bei Raumtemperatur 10 h gerührt. Die Suspension wurde abfiltriert und das Filtrat eingedampft. Der Rückstand wurde in 750 ml Isopropylacetat suspendiert und in Gegenwart von 0,5 g Aktivkohle auf Rückfluss 1,5 h abgekocht. Nach der Aktivkohle-Filtration (70 - 80 °C) wurde das Filtrat über Nacht bei 0 °C gekühlt. Die Titelverbindung wurde filtriert, mit 80 ml kaltem Isopropylacetat gewaschen und unter Vakuum getrocknet. Man erhielt 17,2 g Produkt. Die Ausbeute betrug 66 %.

### 4.3 Herstellung von (1R,4S)-N-Butyryl-1-amino-4-(hydroxymethyl)-2-cyclopenten

34,7 g (1R,4S)-1-Amino-4-hydroxymethyl-2-cyclopenter-hydrochlorid und 2 g N,N-4-Dimethylaminopyridin wurden in 600 ml Methylenchlorid gelöst. Die Lösung wurde auf 0 °C gekühlt. Dann wurden 52 g Triethylamin zugetropft (5 Min.). Das Gemisch wurde noch 30 Min weitergerührt Zu der Mischung wurde bei 0 °C eine Lösung von 35,2 g Butyrylchlorid in 60 ml Methylenchlorid während 1 h zudosiert. Das Gemisch wurde noch zwischen 0 bis 20°C 1,5 h weitergerührt, dann mit 600 ml Wasser versetzt.
Nach Phasentrennung wurde die Wasserphase mit 600 ml Methylenchlorid extrahiert. Die vereinigten organischen Phasen wurden 3mal mit je 500 ml 10 %iger NaOH gewaschen, dann vollständig eingedampft. Der getrocknete Feststoff wurde in 120 ml Methanol gelöst. Die Lösung wurde mit 5 g K₂CO₃ versetzt und bei Raumtemperatur 2 h weitergerührt. Die anorganischen Salze wurden abfiltriert und mit 20 ml Methanol gespült. Das Filtrat wurde mit 2N HCl neutralisiert. Die Suspension wurde abfiltriert und der Filterkuchen mit 20 ml Methanol gewaschen. Das Filtrat wurde vollständig eingedampft. Der feste Rückstand wurde getrocknet und in 150 ml Toluol kristallisiert. Man erhielt 28,5 g Titelverbindung. Die Ausbeute betrug 67 %.

### Beispiel 5: Herstellung von [4(R)-(2-Amino-6-chloropurin-9-yl)cyclopent-2-en-1(S)-yl]methanol

### 5.1 Herstellung von [4(R)-(2-Amino-6-chloropurin-9-yl)cyclopent-2-en-1(S)-yl]methanol ausgehend von 1R,4S-(4-Amino-2-cyclopenten-1-yl)methanol-D-hydrogentartrat

47,4 g 1R,4S-(4-Amino-2-cyclopenten-1-yl)methanol-D-hydrogentartrat (0,18 mol, ee > 98 %) in 200 ml Ethanol wurde vorgelegt. Bei Raumtemperatur wurden 54,6 g NaHCO₃ (0,65 mol) und 37,3 g (0,18 mol) N-(2-Amino-4,6-dichlor-4-pyrimidyl)formamid zugegeben, 9 h unter Rückfluss gekocht und dann auf Raumtemperatur gekühlt. Die Salze wurden abfiltriert und mit 50 ml Ethanol nachgewaschen. Das Filtrat wurde im Rotavapor auf 280 g aufkonzentriert. In der erhaltenen Lösung wurden 18,4 g HCl Gas bei T < 25°C eingeleitet, dann wurden 95,5 g (0,9 mol) Trimethylorthoformiat zugegeben und das Ganze auf 40 °C aufgeheizt (10 min). Bei dieser Temperatur wurde mit Chlorpurin Hydrochlorid angeimpft Nach 2 h bei 42°C kristallisierte das Produkt aus. Die Suspension wurde auf 15 °C gekühlt. Das Produkt wurde filtriert und 3mal mit 50 ml Ethanol nachgewaschen, anschliessend bei 50°C in Vakuum getrocknet. Die Ausbeute betrug 41,9 g (75.8 %). Beiges Pulver, Gehalt (HPLC) : 95.0 %.

### 5.2 Herstellung vou [4(R)-(2-Amino-6-chloropurin-9-yl)cyclopent-2-en-1(S)-yl]-methanol ausgehend von (-)-2-Azabicyclo[2.2.1]hept-5-en-3-on

In einen mit N₂ inertisierten 2 l - Autoklaven (V4A) wurden 61,4 g Natriumborhydrid 97.5% (1,623 Mol), 70,2 g Lithiumchlorid 98,5% (1,656 Mol), 13,2 g Celite sowie 1410 g Tetrahydrofuran eingefüllt. Der Autoklav wurde verschlossen, es wurde auf eine Innentemperatur von 130°C aufgeheizt und 4,5 Stunden bei dieser Temperatur gerührt (max. 8,0 bar). Nach Abkühlen auf ca. 60 °C wurden die in Tetrahydrofuran unlöslichen Natriumsalze (NaCl, NaBH₄) abfiltriert. Diese wurden mit 353 g Tetrahydrofuran gewaschen und die vereinigten Filtrate wurden in einem 1 1-Glasrührwerk durch Destillation bei Normaldruck auf ca die Hälfte eingeengt (Destillat 1: ca. 710 g Tetrahydrofuran). Anschliessend wurde durch weitere Destillation, im Wechsel mit portionsweiser Zugabe von insgesamt 936 g Dioxan der Lösungsmitteltausch vollendet (Destillat 2: ca. 1289 g Tetrahydrofuran/Dioxan).
Zur auf ca. 60 °C gekühlten LiBH₄-Suspension wurden 56,7 g (-)-2-Azabicyclo[2.2.1]hept-5-en-3-on (97,5% / 0,507 Mol) addiert. Bei ca. 60 °C beginnend wurden in exakt einer Stunde 132,5 g Methanol so zudosiert, dass ein Temperaturbereich von 58 - 62 °C eingehalten wurde. Es wurde eine weitere Stunde bei 60 °C nachreagieren gelassen. Anschliessend wurden weitere 397,0 g Methanol addiert (Probe enthält eine analytische Ausbeute von 70,5%) und der Rührwerksinhalt wurde auf 0 °C abgekühlt. Bei dieser Temperatur wurden 90,0 g HCl in die Reaktionsmischung eingeleitet (leicht exotherm) und es wurde eine weitere Stunde bei ca 0 °C gerührt. Die Lösung wurde am Rotavapor bei 50° C unter Vakuum eingeengt, 200 ml Methanol wurden zugesetzt und das Methanol wurde wieder entfernt (absaugen des Methylborates). Vorgang wurde mit weiteren 200 ml Methanol wiederholt. Das erhaltene Oel (253,4 g enthalten 3,16 % Aminoalkohol dies entsprach 0,360 Mol) wurde mit 250 ml Ethanol versetzt und in einem 11-Doppelmantelrührgefäss gegeben. Bei Raumtemperatur 72,6 g NaHCO₃ (0,86 Mol) und 74,6 g (0,360 Mol) N-(2-Amino-4,6-dichlor-4-pyrimidyl)formamid zugeben, 9 h wurde rückflussiert, auf Raumtemperatur gekühlt, Salze abfiltriert und mit 100 ml Ethanol nachwaschen.
Das Filtrat im Rotavapor wurde auf 560 g aufkonzentriert. In der erhaltenen Lösung wurden 63,4 g HCl Gas bei T < 25°C eingeleitet, dann 191,0 g (1,80 Mol) Trimethylorthoformiat zugeben, auf 40 °C aufgeheizt (10 min). Bei dieser Temperatur mit Chlorpurin Hydrochlorid angeimpft, 2 h bei 42 °C wurde kristallisiert getassen. Die Suspension wurde auf 15 °C gekühlt. Das Produkt wurde filtriert und mit 3-mal 50 ml Ethanol nachgewaschen, anschliessend bei 50 °C i. V. getrocknet. Die Ausbeute betrug 66,0 g (59,7 %). Beiges Pulver, Gehalt(HPLC): 89,3 %
Dies entsprach einer Ausbeute von 42,4 % bzgl. eingesetztem Vince-Lactam.

## Patentansprüche

1. Verfahren zur Herstellung von (1S,4R)- oder (1R,4S)-1-Amino-4-(hydroxymethyl)-2-cyclopenten der Formeln 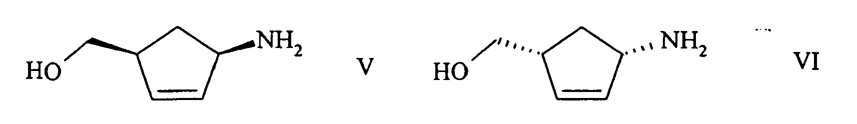 bzw. deren Salzen, umfassend die Racematspaltung eines racemischen Aminoalkohols der Formel 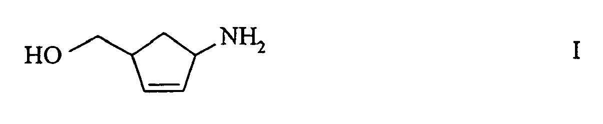 mittels einer optisch aktiven Weinsäure.

2. Verfahren nach Anspruch 1, **dadurch gekennzeichnet, dass** die Racematspaltung mit D-(-)- oder L-(+)-Weinsäure durchgeführt wird.

3. Verfahren zur Herstellung von (1R,4S)- oder (1S,4R)-1-Amino-4-(hydroxymethyl)-2-cyclopentenderivaten der allgemeinen Formeln 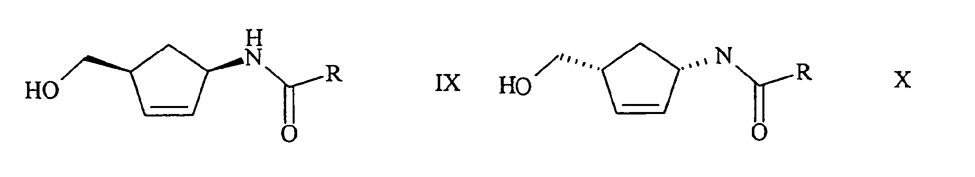 worin R C₁₋₄-Alkyl, C₁₋₄-Alkoxy, Aryl oder Aryloxy bedeutet, **dadurch gekennzeichnet, dass** man in einer ersten Stufe (±)-2-Azabicyclo[2.2.1]hept-5-en-3-on der Formel 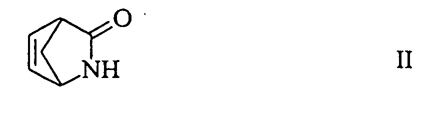 in Form des Racemats oder eines seiner optisch aktiven Isomeren mit einem Metallhydrid zu einem racemischen Aminoalkohol der Formel 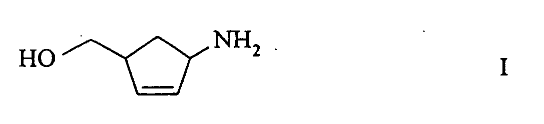 reduziert, diesen in einer zweiten Stufe mit einer optisch aktiven Weinsäure zum (1S,4R)- oder (1R,4S)-1-Amino-4-(hydroxymethyl)-2-cyclopenten der Formel V oder VI umsetzt und diese zu den Produkten der Formeln IX oder X acyliert.

4. Verfahren zur Herstellung von (1S,4R)- oder (1R,4S)-4-(2-amino-6-chlor-9-H-purin-9-yl)-2-cyclopenten-1-methanol, oder dessen Salzen, der Formeln 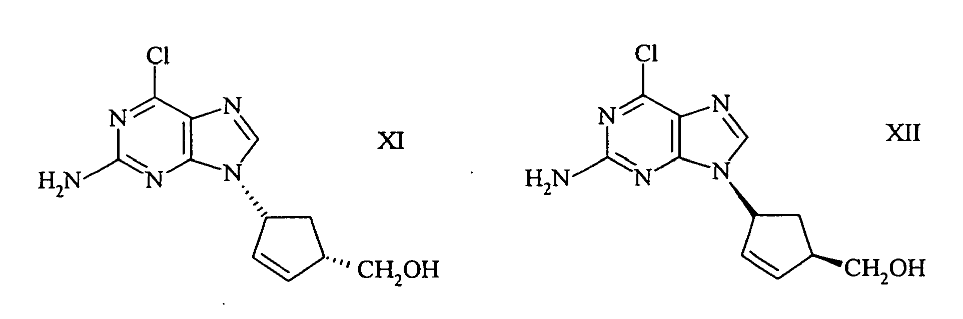 **dadurch gekennzeichnet, dass** man (1 R,4S)- oder (1S,4R)-1-Amino-4-(hydroxymethyl)-2-cyclopentenhydrogen-D- oder L-tartrat mit N-(2-amino-4,6-dichlorpyrimidin)-5-yl)formamid der Formel 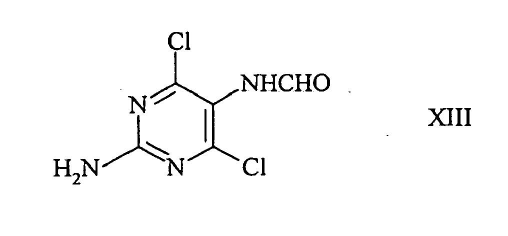 in das (1S,4R)- oder (1R,4S)-4-[(2-amino-6-chlor-5-formamido-4-pyrimidinyl)-amino]-2-cyclopenten-1-methanol der Formeln 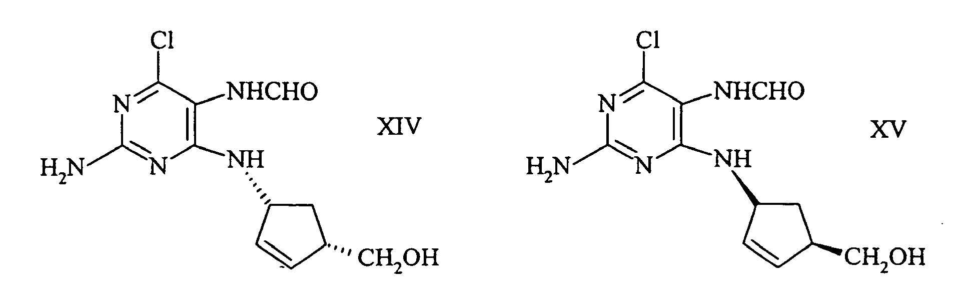 überführt und letzteres dann auf bekannte Weise zu den Verbindungen gemäss Formel XI oder XII cyclisiert.

5. (1R,4S)-1-Amino-4-(hydroxymethyl)-2-cyclopenten-hydrogen-D- oder L-tartrat

6. (1S,4R)-1-Amino-4-(hydroxymethyl)2-cyclopenten-hydrogen-L- oder D-tartrat.

## Claims

1. Process for preparing (1S,4R)- or (1R,4S)-1-amino-4-(hydroxymethyl)-2-cyclopentene of the formulae 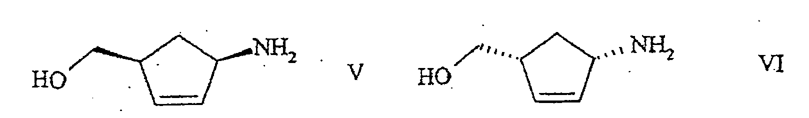 or the salts thereof, comprising the racemate resolution of a racemic amino alcohol of the formula 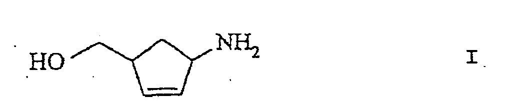 using an optically active tartaric acid.

2. Process according to Claim 1, **characterized in that** the racemate resolution is carried out with D-(-)- or L-(+)-tartaric acid.

3. Process for preparing (1R,4S)- or (1S,4R)-1-amino-4-(hydroxymethyl)-2-cyclopentene derivatives of the general formulae 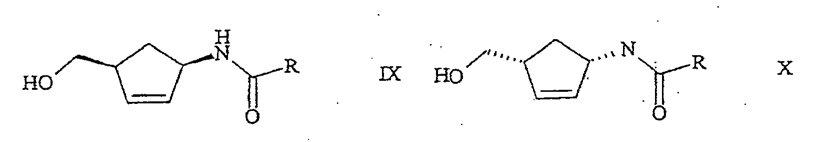 in which R is C₁₋₄-alkyl, C₁₋₄-alkoxy, aryl or aryloxy, **characterized in that** in a first stage (±)-2-azabicyclo[2.2.1]hept-5-en-3-one of the formula 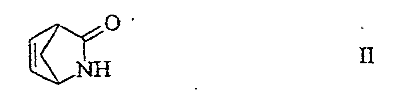 in the form of the racemate or of one of its optically active isomers is reduced with a metal hydride to a racemic amino alcohol of the formula 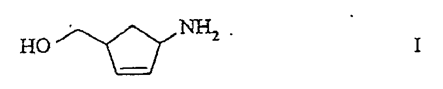 the latter is reacted in a second stage with an optically active tartaric acid to give the (1S,4R)- or (1R,4S)-1-amino-4-(hydroxymethyl)-2-cyclopentene of the formula V or VI, and the latter is acylated to give the products of the formulae IX or X.

4. Process for preparing (1S,4R)- or (1R,4S)-4-(2-amino-6-chloro-9-H-purin-9-yl)-2-cyclopentene-1-methanol, or the salts thereof, of the formulae 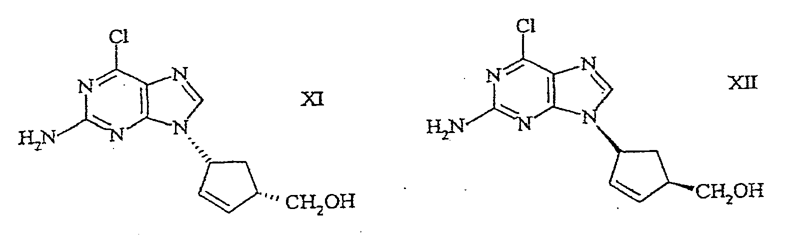 **characterized in that** (1R,4S)- or (1S,4R)-1-amino-4-(hydroxymethyl)-2-cyclopentene hydrogen D- or L-tartrate is converted with N-(2-amino-4,6-dichloropyrimidin)-5-yl)formamide of the formula 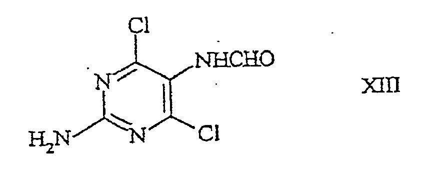 into the (1S,4R)- or (1R,4S)-4-[(2-amino-6-chloro-5-formamido-4-pyrimidinyl)-amino]-2-cyclopentene-1-methanol of the formulae 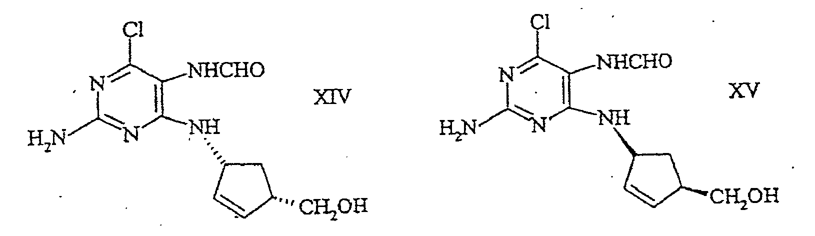 and the latter is then cyclized in a known manner to give the compounds of formula XI or XII.

5. (1R,4S)-1-Amino-4-(hydroxymethyl)-2-cyclopentene hydrogen D- or L-tartrate.

6. (1S,4R)-1-Amino-4-(hydroxymethyl)-2-cyclopentene hydrogen L- or D-tartrate.

## Revendications

1. Procédé de préparation du (1S,4R)- ou du (1R,4S)-1-amino-4-(hydroxyméthyl)-2-cyclopentène de formules 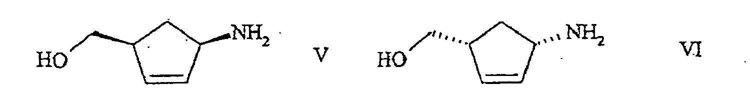 ou de leurs sels, comprenant la dissociation du racémate d'un amino-alcool racémique de formule 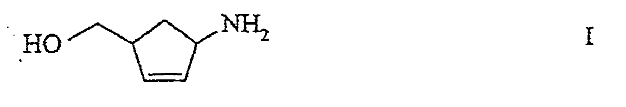 à l'aide d'un acide tartrique optiquement actif.

2. Procédé selon la revendication 1, **caractérisé en ce que** la dissociation du racémate est réalisée avec l'acide D-(-)- ou L-(+)-tartrique.

3. Procédé de préparation de dérivés du (1R,4S)- ou du (1S,4R)-1-amino-4-(hydroxyméthyl)-2-cyclopentène de formules générales 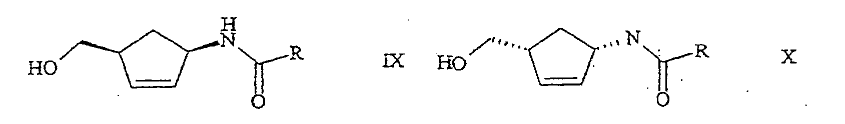 dans lesquelles R représente un alkyle en C₁₋₄, un alcoxy en C₁₋₄, un aryle ou un aryloxy, **caractérisé en ce que**, dans une première étape, la (±)-2-azabicyclo[2.2.1]hept-5-én-3-one de formule 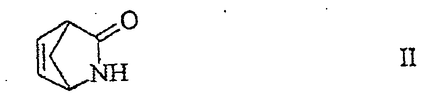 sous forme du racémate ou de l'un de ses isomères optiquement actifs, est réduite avec un hydrure métallique en un amino-alcool racémique de formule 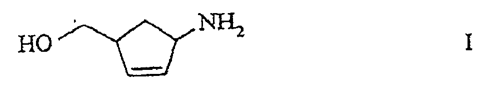 dans une deuxième étape, celui-ci est mis à réagir avec un acide tartrique optiquement actif pour donner lieu au (1S,4R)- ou au (1R,4S)-1-amino-4-(hydroxyméthyl)-2-cyclopentène de formule V ou VI, et celui-ci est acylé pour donner lieu au produit de formule IX ou X.

4. Procédé de préparation du (1S,4R)- ou du (1R,4S)-4-(2-amino-6-chloro-9-H-purin-9-yl)-2-cyclopentène-1-méthanol, ou de ses sels, de formules 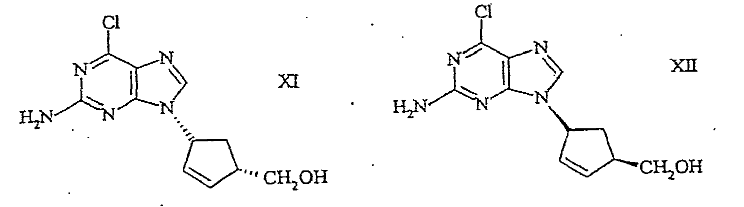 **caractérisé en ce que** le (1R,4S)- ou (1S,4R)-1-amino-4-(hydroxyméthyl)-2-cyclopentène-hydrogéno-D- ou L-tartrate est transformé, avec le N-(2-amino-4,6-dichloropyrimidin)-5-yl)formamide de formule 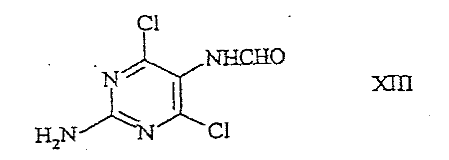 en le (1S,4R)- ou le (1R,4S)-4-[(2-amino-6-chloro-5-formamido-4-pyrimidinyl)-amino]-2-cyclopentène-1-méthanol de formules 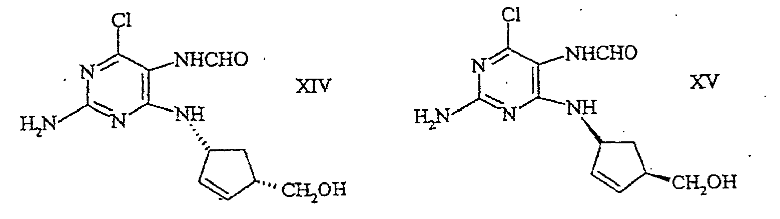 et ce dernier est alors cyclisé de façon connue pour donner lieu aux composés selon la formule XI ou XII.

5. (1R,4S)-1-amino-4-(hydroxyméthyl)-2-cyclopentène-hydrogèno-D- ou L-tartrate.

6. (1S,4R)-1-amino-4-(hydroxyméthyl)-2-cyclopentène-hydrogèno-L- ou D-tartrate.
